# EUROPEAN PATENT APPLICATION

(11) **EP 3 292 809 A2**
(43) Date of publication of application: **14.03.2018**
(21) Application number: 16726617.0
(22) Date of filing: 03.05.2016
(51) Int. Cl.: A61B 1/00, A61B 1/005

(54) **ANGULATION DEVICE FOR AN ENDOSCOPE, CONTROL DEVICE FOR THE PERIPHERAL ELEMENTS OF AN ENDOSCOPE, AND AN ENDOSCOPE PULSE UNIT**

(30) Priority: 06.05.2015 ES 201530615
(71) Applicant: Soriano Romero, Francisco Santiago, 08041 Barcelona (ES)
(72) Inventor: Soriano Romero, Francisco Santiago, 08041 Barcelona (ES)
(74) Representative: Isern-Jara, Nuria
(86) International application number: PCT/ES2016/070338
(87) International publication number: WO 2016/177925

(57) **Abstract**

An angulation device for a flexible-type endoscope comprising an angulation body with a first angulation portion linked to a second angulation portion, said first and second angulation portion being articulated and defining a relative back-and-forth movement therebetween, causing a bending portion of the endoscope to be angled; it being possible for said angulation device to be in combination with a control device for peripheral elements of an endoscope comprising a plurality of electromyography sensors and another control device comprising a plurality of buttons arranged around a crown; it being possible for said devices to be in combination with an endoscopy supply tower that comprises processing means and at least one peripheral element, and an output linked to the peripheral element and said output being connectable to a conduit of the endoscope linked to an insertion tube.

## Description

### OBJECT OF THE INVENTION

The aim of the present application is to register an angulation device for a flexible endoscope, a control device for the peripheral elements of said endoscope and a supply tower that includes significant innovations.

More specifically, the invention proposes the development of an angulation device that enables the use of conventional movement wheels to be removed, as well as a new control device for the peripheral elements of the endoscope and a supply tower that enables the body of the endoscope to be free of valves and conduits.

### BACKGROUND OF THE INVENTION

The flexible endoscope is a medical probe for exploring the cavities of the human body, which is inserted through the orifices and sphincters of the human body. Said apparatus has been used since the end of the 1950s and its format has remained largely unchanged.

The only significant change since its creation was at the beginning of the 1990s, when the optical fibre was replaced by a micro-camera in order to display the image on a monitor. Until then, the size of the cameras meant they had to be placed on the outside.

There is very little variation in the format of the endoscope body between the oldest models and the current ones, apart from where there was a lens, there is now a button to remotely control the image processor, and that now the connector between the endoscope and the control elements thereof needs an electronic connection for the camera.

This endoscope format, with slight aesthetic modifications, is the format that all endoscope manufacturers are currently using because they all share the same system to angle the bending section.

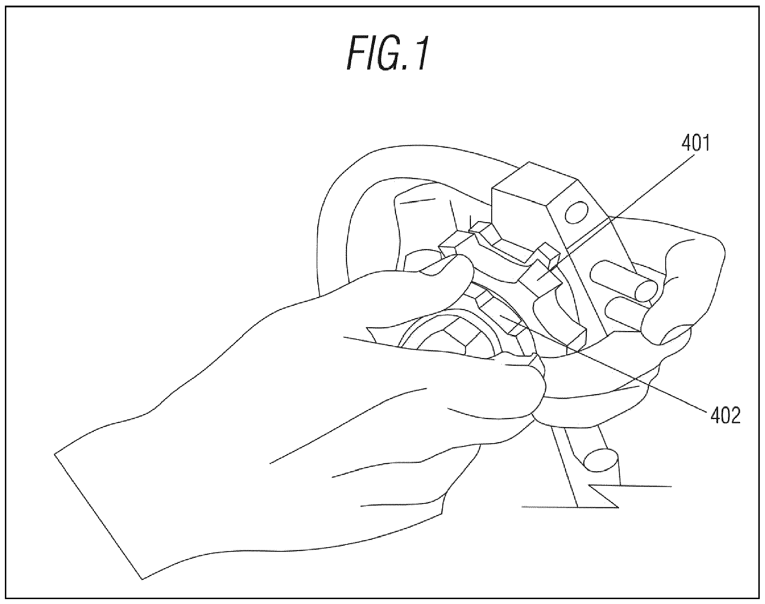

As may be seen in figure 1, the current system to angle the bending section comprises, for example, a pair of wheels 401, 402, which serve as controls, in order to turn the pulleys, or gear wheels, that move a chain or cable which pulls on the cables of the bending portion. These cables are inside a spring, which passes through the entire insertion tube, so that the movement is only possible in the bending section. The ends of the cables are fastened to the distal end of the bending portion.

The bending portion is a piece made up of a number of metal rings arranged as a tube, which may be angled in four directions in order to guide the endoscope through the inside the human body.

This angulation system has always been used since the first models. This has a first drawback: the number of pieces needed to fulfil this function.

Another drawback is the poor ergonomics of the current endoscope configuration. The wheel must be turned using the end of the fingers of the left hand, which holds the endoscope, whilst the other wheel is turned by the right hand.

In addition, if operations such as cleaning the lens, pumping gas into the body of the patient, liquid suction, or taking a photograph of the image with an image processor, etc (peripherals) must be carried out, one of the buttons on the upper portion of the casing, where the wheels are also situated, has to be pressed. This entails having to release the wheel, meaning the brake has to be applied, or stretching the fingers to the other end of the casing.

Factors such as people with smaller hands than others, which means it is very hard for their fingers to easily span the casing, must be taken into account. Moreover, all endoscopes are made to be used by right-handed users, given that this is the hand left "free" (after having applied the brake) in order insert accessories through the channel of the endoscope, which is a significant disadvantage for left-handed users.

Another drawback of the state of the art is that the body of the endoscope is equipped with valves, interconnections and other types of commutations that may cause fluid leaks and growth of toxic bacteria, fungi or viruses.

### DESCRIPTION OF THE INVENTION

The present invention has been developed with the aim of providing an angulation device for an endoscope and a control device for the peripheral elements of the endoscope that resolve the disadvantages mentioned above while also contributing other additional advantages, which will become evident from the description provided below.

An object of the present invention is an angulation device for a flexible-type endoscope comprising a bending portion and bending cables covered by covers, comprising an angulation body with a long configuration and a central axis arranged longitudinally along the length of said angulation body, wherein the angulation body in turn comprises a first angulation portion linked to a second angulation portion, said first and second angulation portion being articulated such that a relative back-and-forth movement therebetween is defined with regard to the central axis, at least one first bending cable being fastened to the first angulation portion and at least one first cover fastened to the second angulation portion, such that the relative back-and-forth movement between the first angulation portion and the second angulation portion causes the relative movement of the first bending cable with respect to the first cover.

Thanks to these characteristics, an angulation device is achieved that may be used regardless of the hand size of the user and whether they are right-handed or left-handed. The angulation may be achieved with either hand, something which is not currently possible with the movement wheels. Furthermore, it is not necessary for the user to stop the endoscope when the peripherals must be operated. Ultimately, the ergonomics are greatly optimised; the angulation body only needs to be "bent" in order for the distal end of the endoscope to be bent (bending portion).

Another advantage of the present invention is that the angulation system of the endoscope is greatly simplified, thus making the manufacture, maintenance and disinfection thereof much easier, as well as the decrease in technical faults due to most of the pieces wearing in comparison to the models of the state of the art.

To make the invention easy to handle, the angulation body may further comprise a casing that covers the different angulation portions, said casing having a first end by way of a handle. The casing may further comprise a second end opposite the first end, said second end being provided with at least one pair of protuberances that are divergent from one another, from one of which the covers and the bending cables extend.

In order to comfortably hold the casing, the present angulation device for an endoscope may further comprise a support element that in turn comprises at least one support surface configured to coincide at least partially with the casing and a projection that may fit between the pair of divergent protuberances when in use. This support element may be linked to a stand, which may optionally be height-adjustable. This support element thus enables the comfortable angulation of several movements with a single hand.

Advantageously, the present angulation device for an endoscope may further comprise at least one third angulation portion linked to the second angulation portion, said second and third angulation portion being articulated such that a relative back-and-forth movement therebetween is defined with respect to the central axis, at least one second bending cable being fastened to the second angulation portion and at least one second cover fastened to the third angulation portion, such that the relative back-and-forth movement between the second angulation portion and the third angulation portion causes the relative movement of the second bending cable with respect to the second cover. In this way, by being able to add as many angulation portions as necessary, angulations with more accurate direction changes may performed given that these additional angulation portions may be joined to bending cables that perform additional changes.

Thus, an additional advantage of the present design lies in the simplicity of use that enables, for example, two movements to be carried out simultaneously, faster and more easily than can be done using an endoscope with movement wheels.

In accordance with another embodiment, the angulation portions may comprise hollow tubes, a pair of angulation portions being linked to each other with rods arranged inside the angulation portions, the ends of said rods being connected to the respective angulation portions such that they rotate with respect to each other. Preferably, the hollow tubes may have a square transverse cross section.

Alternatively, the angulation portions may be linked to each other with a spherical or ball joint that may be advantageously limited to one degree of freedom. This limitation means that the relative movement between two angulation portions only entails the modification in one direction of the endoscope, thus facilitating intuitive handling by the user.

The covers may advantageously be springs, since they protect the bending cables and have a certain degree of flexibility in order to adopt the changes in angulation of the endoscope.

Another object of the present invention is a control device for the peripheral elements of an endoscope comprising a plurality of electromyography sensors arranged on a band made of elastic material, particularly configured as a bracelet or similar, wherein the electromyography sensors may establish data communication with processing means and are in turn linked in data communication with at least one peripheral element.

In the present invention, the expression peripheral elements must be understood as the auxiliary elements used to carry out the endoscopy, such as the light source, water source, blowing, suction, camera, etc. which in the present invention are advantageously situated in a supply tower linked to the endoscope body itself. The supply tower is the body that supports these peripheral elements comprising a valve or switch that manages the activation thereof.

The band may preferably be made of elastic material, which enables it to be optimally adapted to the anatomy of the user.

Advantageously, the casing described above may comprise predetermined marks situated at the first end by way of a handle, which advantageously may be recesses or reliefs. Due to these predetermined marks, it is easier for the user to place the fingers on the angulation body in a specific way, as if to press a virtual button, in order to generate a series of electrical pulses at specific points of the muscles that are measured by the electromyography sensors and interpreted by the processing means as a predefined order to operate certain peripheral elements.

Alternatively, the control device for the peripheral elements of an endoscope comprises a plurality buttons arranged around a crown, wherein the buttons of the crown may establish data communication with processing means and are in turn linked in data connection with at least one peripheral element.

These characteristics achieve a pair of control devices for peripheral elements preferably and advantageously in cooperation with the angulation device describe above in order to perform an endoscopy in an ergonomic manner. The user may hold the angulation body with two hands or even just one, and can handle the control device that operates the peripheral elements of the endoscope through commutation means without letting go of said angulation body. The processing means receive data from the electrical muscular impulses and interpret the action to be carried out.

Advantageously, the crown may be configured such that a link with a first end of the casing may be established by way of male-female coupling. This makes the angulation body even easier to handle given that the angulation body may be inserted through the inner hole of the crown.

The crown may then be used as a removable control, with physical buttons and be used second-hand in another endoscope that is not the original one.

Another object of the present invention is a supply tower for an endoscope that comprises processing means and at least one peripheral element, wherein the peripheral element in turn comprises commutation means linked to the processing means, further comprising at least one output associated with the peripheral element and said output being connectable to a conduit of the endoscope linked to an insertion tube; wherein the processing means are able to be linked in data communication with a control device for peripheral elements.

These characteristics free the endoscope body from valves, pistons and breakers, which are needed for sending and returning fluids, image transmission or the light source. Multiple interconnections of tubes and metal pieces that are a special risk, given that they are areas that are hard to clean and disinfect, are removed.

In the present invention, the supply tower may adopt any outside configuration, for example a cube, prism, cylinder, etc. Advantageously, the conduit may also link the output to the other projection of the angulation device for an endoscope as defined above.

Regarding the commutation means, they may comprise a peristaltic pump, a solenoid valve or switch, which are necessary to allow or block the passage of fluids, the transmission of light or images.

Other characteristics and advantages of the angulation device for an endoscope, the control device for peripheral elements of the endoscope and the supply tower, the subject matter of the present invention, will become clear in light of the description of the preferred, though non-exclusive, embodiments, which, by way of a non-limiting examples, are illustrated in the accompanying drawings, wherein:

### BRIEF DESCRIPTION OF THE DRAWINGS

Figure 1 is a schematic perspective view of an angulation and control device for an endoscope of the state of the art;
Figure 2 is a schematic perspective and partial cross-sectional view of a first embodiment of an angulation and control device according to the invention in a first position;
Figure 3 is a schematic perspective and partial cross-sectional view of a first embodiment of an angulation and control device according to a second position;
Figure 4 is a schematic perspective and partial cross-sectional view of a second embodiment of an angulation device according to the invention;
Figure 5 is a schematic elevation view of an angulation body with a plurality of predefined marks corresponding to a first embodiment of the control device;
Figure 6 is a schematic elevation view of an angulation body with a fitted crown corresponding to a second embodiment of the control device;
Figure 7 is a schematic plan view of the angulation body and the crown in figure 6; and
Figure 8 is a schematic view of an endoscope with an angulation device, a control device and a supply tower according to the present invention.

### DESCRIPTION OF A PREFERRED EMBODIMENT

In light of the aforementioned figures and in accordance with the adopted numbering, one may observe therein examples of preferred embodiments of the invention, which comprises the parts and elements that are indicated and described in detail below.

Some elements that cannot be seen have been represented with dashed lines for the purposes of clarity.

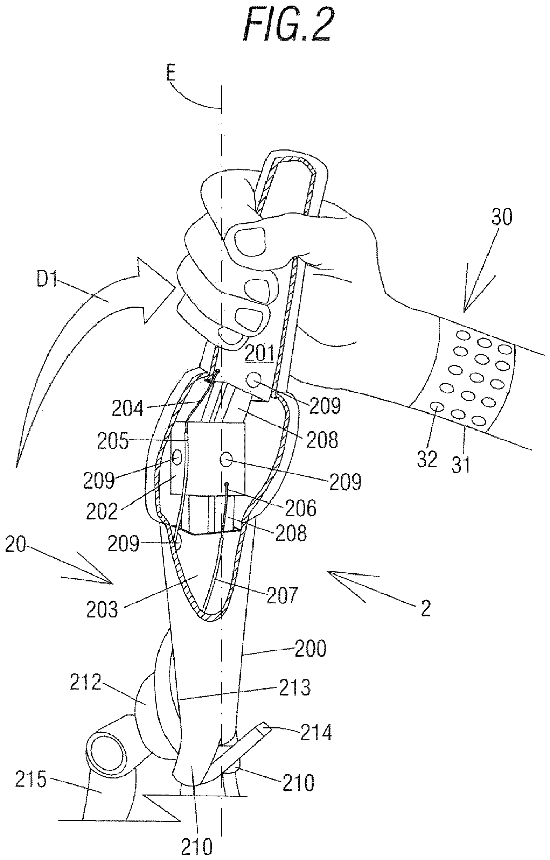

In figures 2 - 8 an angulation device 2 for a flexible endoscope 1 may be seen which comprises a bending portion 5 and bending cables 204, 206 covered by covers 205, 207. This bending portion 5 of the endoscope shall not be described in more detail since it is of the type already available on the market.

Continuing with the description, the angulation device 2 comprises an angulation body 20 with a long configuration and a central axis E arranged longitudinally along the length of said angulation body 20. The angulation body 20 in turn comprises a first angulation portion 201 linked to a second angulation portion 202, said first and second angulation portion 201, 202 being articulated such that a relative back-and-forth movement therebetween is defined with respect to the central axis E.

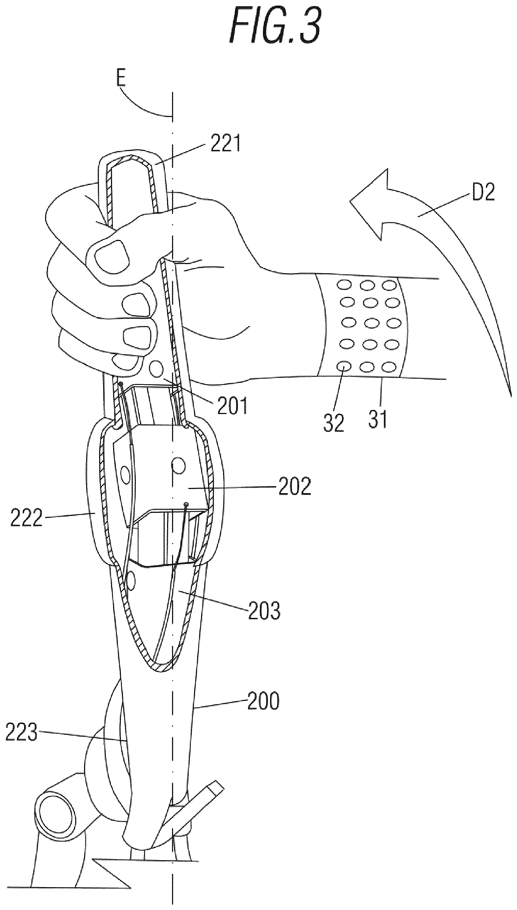

Said back-and-forth movement may be more clearly seen in figures 2 and 3; one may observe therein how the first and second angulation portion 201, 202 are linked to each other such that a relative back-and-forth movement therebetween is enabled. The position of part of the angulation body 20 varies with respect to the longitudinal axis E. The joints provided will be described below.

A first bending cable 204 is fastened to the first angulation portion 201 and a first cover 205 is fastened to the second angulation portion 202, such that the relative back-and-forth movement between the first angulation portion 201 and the second angulation portion 202 causes the relative movement of the first bending cable 204 with respect to the first cover 205 in order to accurately angle the bending portion 5.

Despite the fact that the present description only mentions a single bending cable, it must be understood that it is preferred for said bending cables to be in pairs as may be seen in figures 2, 3 and 4 and more preferably, arranged diametrically opposite the longitudinal axis E.

Continuing with figures 2 and 3, it may be seen that the angulation body 20 preferably comprises a casing 200 that covers the different angulation portions 201, 202, said casing 200 having a first end 221 by way of handle. The casing 200 may further comprise a second end 223 opposite the first end, and this second end being preferably provided with at least one pair of protuberances 210 that are divergent from one another, and from at least one of which the covers 205, 207 and the bending cables 204, 206 extend. It is obvious that the number of protuberances 210 may vary depending on the bending cables included in the invention or on other connections that are to be provided. It may also be seen that a central portion 222 has been arranged between the first and second end 221, 223 in order to send the aforementioned relative back-and-forth movements.

The casing 200 may be made from a plastic material that is suitable for use in medicine. Preferably, the first and second end 221, 223 and the central portion 222 are joined to each other in a sealed manner for hygiene purposes.

The present angulation device 2 further comprises a support element 212 in turn comprising a support surface 213 configured to coincide at least partially with the casing 200, i.e. that the support surface 213 has a configuration that is complementary with a portion of the casing 200 in order to achieve a suitable connection therebetween. The support element 212 further comprises a projection 214 that may fit between the pair of divergent protuberances 210 when in use, as shown in figures 2 and 3. In this way, the user may handle the angulation device 2 with a single hand, leaving the other one free.

Depending on the particular needs, the support element 212 may be linked to a stand 215 and even to a rotating attachment point 216 that enables the height and tilt to be adjusted to the physical characteristics of the user of the endoscope 1, thus facilitating even more if possible the handling ergonomics.

In figures 2 and 3 it may be seen that the angulation device 2 additionally comprises at least one third angulation portion 203 linked to the second angulation portion 202, said second and third angulation portion 202, 203 being articulated such that a relative back-and-forth movement therebetween is defined with respect to the central axis E. This articulation is identical to the one described for the first and second angulation portion 201, 202 and has the same technical effect. However, the relative back-and-forth movement between the second and third angulation portion 202, 203 is produced in a different direction to that of the relative back-and-forth movement between the first and second angulation portion 201, 202. The second bending cable 206 is fastened to the second angulation portion 202 and the second cover 207 is fastened to the third angulation portion 203, such that the relative back-and-forth movement between the second angulation portion 202 and the third angulation portion 203 causes the relative movement of the second bending cable 206 with respect to the second cover 207.

As has already been mentioned, the relative back-and-forth movement is produced in a different direction with respect to the longitudinal axis E for each articulation, modifying in this way the angulation of the bending portion 5 in more directions. That is, that as the number of angulation portions 201, 202, 203 increases, so do the number of possibilities for varying the angle of the bending portion 5 of the endoscope 1.

The bending cables 204, 206 and the covers 205, 207 may be fastened to the respective angulation portions 201, 202, 203 via any known means, such as welding, screw elements, rivets, etc. Preferably both the bending cables 204, 206 and the covers 205, 207 are metal and specifically the covers 205, 207 are springs. However, it is obvious for a person skilled in the art to modify the materials and adapt the configuration of these elements to suit the particular needs.

In accordance with a preferred first embodiment of the angulation device 2, shown in figures 2 and 3, the angulation portions 201, 202, 203 comprise hollow tubes, a pair of angulation portions being linked to each other with rods 208, the ends of which are arranged inside the angulation portions 201, 202, 203, the ends of said rods 208 being connected to the angulation portions 201, 202, 203 such that they rotate with respect to each other. Even more preferably, the hollow tubes have a square transverse cross section to make it easier to position the bending cables 204, 206.

In the present embodiment it has been provided for that the rods 208 are arranged in pairs for greater strength although it is obvious that the number may vary. These pairs of rods 208 may be joined to the hollow tubes by means of pins 209, for example, that enable the relative rotation of the angulation portions 201, 202, 203 with respect to each other. As may be seen in figures 2 and 3, the pins 209 are arranged in twos for each joint, such that the two pairs of pins 209 have a different and displaced location to each other, with respect to the longitudinal axis E. This enables the relative back-and-forth movement between the second and third angulation portion 202, 203 in a direction that is different to the relative back-and-forth movement between the first and second angulation portion 201, 202.

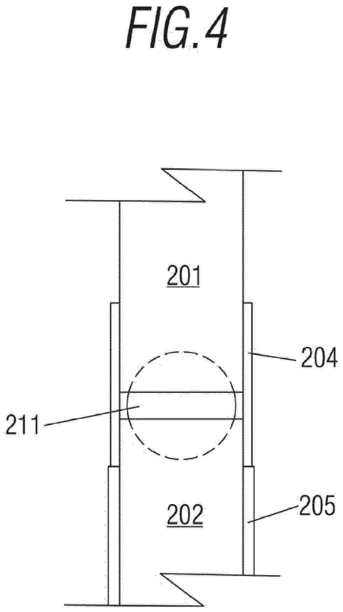

Figure 4 shows a second embodiment of the angulation device 2 wherein the angulation portions 201, 202, 203 are linked to each other with a spherical or ball joint 211. In order to achieve a predictable and controlled back-and-forth movement between the angulation portions 201, 202, 203, the spherical or ball joint 211 may be advantageously limited to one degree of freedom. Another alternative embodiments may include springs (not shown) positioned between the angulation portions 201, 202, 203, which are also limited to one degree of freedom.

Although joints between the angulation portions 201, 202, 203 with only one degree of freedom are preferred in the present description due to the simplicity in handling that is achieved, using joints that allow for more degrees of freedom between the angulation portions 201, 202, 203 and thus achieve relative movements therebetween in more directions with respect to the longitudinal axis E is obvious.

In another object of the invention, a control device 3 for the peripheral elements 72 of an endoscope 1 is described. In accordance with a first embodiment of the control device 3 shown in figures 2, 3 and 5, the control device 3 comprises a plurality of electromyography sensors 32 arranged on a band 31 made of elastic material particularly configured as a bracelet 30 or similar. This bracelet 30 is intended for any part of the arm or the user that handles the endoscope 1; that is, it may be positioned for example on the upper extremity that corresponds to the hands being used. The material of the band 31 may be any that is suitable, preferably elastic, since the bracelet 30 is secured regardless of the user's anatomy.

The electromyography sensors 32 may establish data communication with processing means 71 arranged in a supply tower 7 and linked in data communication to at least one peripheral element 72 arranged in a supply tower 7.

The electromyography sensors 32 may be any within the reach of the person skilled in the art, and be suitably arranged in predetermined locations to measure electrical impulses from the user's muscles. The data communication between the electromyography sensors 32 and the processing means 7 may be carried out wirelessly, for example, since this prevents the presence of cables and connections that would hinder the activity of the specialised personnel.

The peripheral elements 72 may be any of those mentioned above in this specification and thanks to the present invention they may be managed within the supply tower 7. With regards to the processing means 71, they may comprise a processor or similar.

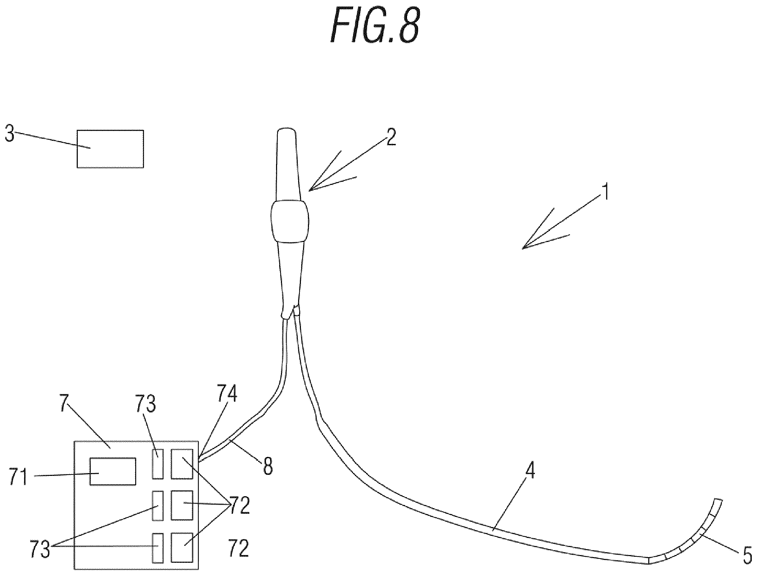

Figure 8 schematically shows a supply tower 7 that comprises the processing means 71 and a plurality of peripheral elements 72. The peripheral element 72 in turn comprises commutation means 73 linked to the processing means 71. The supply tower 7 further comprises an output associated with the peripheral element 72; it being possible to have several outputs 74 associated respectively to each peripheral element 72.

These commutation means 73 may in turn comprise a peristaltic pump, a solenoid valve, a switch, etc. Preferably, the conduit 8 links the output 74 to the other projection 210 of the angulation device 2.

Unlike the known systems, in the present invention the pistons or taps (not shown) for fluids such as water or gas may be situated in the supply tower 7 itself, avoiding the presence of said valves and connections in the body of the endoscope 1 itself (angulation device 2, control device 3, conduit 8, insertion tube 4 or bending portion 5) and thus reducing the complexity thereof and the risk of leaks in said endoscope 1. Another problem that is resolved is that the formation of "biofilm", which feeds bacteria, is prevented.

Through the output 74 that connects the conduit 8 to the supply tower 7, the fluid required by the user is sent, for example, to the insertion tube 4 of the endoscope 1. The supply tower 7 becomes the place where the operation of the different peripheral components of the endoscope 1 is managed, except the angulation which is the responsibility of the angulation device 2. In order to manage said operation, the peripheral devices 72 preferably comprise commutation means 73 that control the passage of fluid to the distal end of the endoscope 1 to be inserted in the patient. The water irrigation may be managed via a peristaltic pump situated in the supply tower 7, which may in turn be connected to a sterilised water supply source.

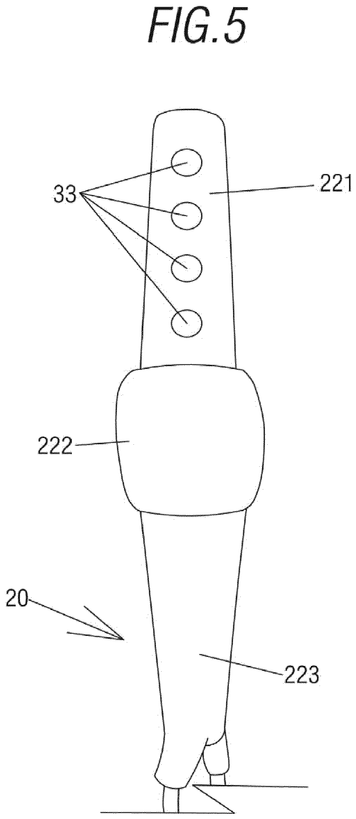

Returning to the first embodiment of the control device 3, and referring to figure 5, on the angulation body 20, specifically the casing 200, there may be predetermined marks 33 situated at the first end 221 by way of handle. These predetermined marks 33 may be recesses or reliefs, and help the user position their fingers in the correct position in order for their monitored muscles, by means of the electromyography sensors 32, to generate certain electrical impulses (not shown) that are associated to specific orders stored by the processing means 71.

In alternative constructions to this first embodiment of the control device 3, not shown, user eye tracking and monitoring systems may be established. An order to be executed by the supply tower 7 may be selected via the orientation of the eyes whilst the hand or hands handle the angulation device 2. These systems may comprise any sensing element of the user eye positioning. In another alternative, the user may handle the angulation device 2 with one hand and with the other arm perform gestures that are detected, for example, via cameras that recognise specific gestures that are compared by the processing means 71 to predetermined gesture patterns associated to specific orders stored in the supply tower 7.

In another alternative that is not shown, the control device 3 may include a capturing and recognition system of voice commands, which are sent to the processing means 71, which in turn send the instruction to the corresponding commutation means 73 in order for the selected peripheral element 72 to execute the action.

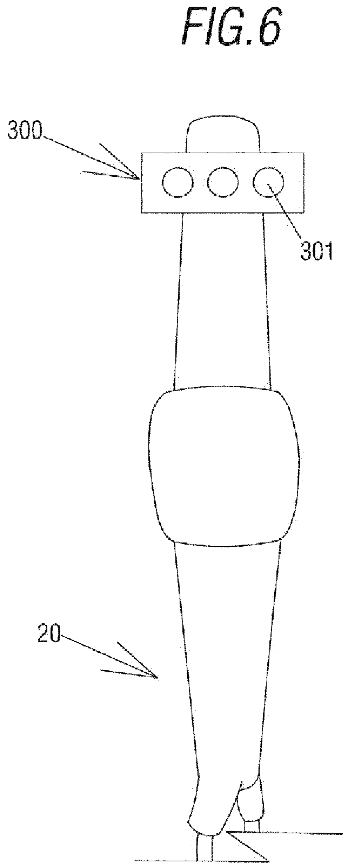

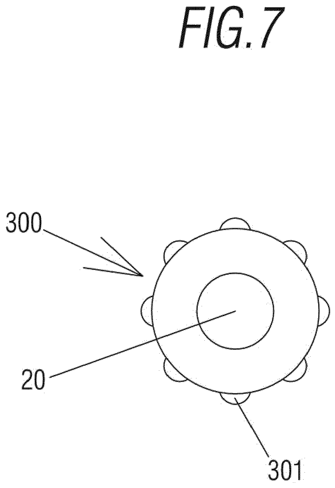

In a second embodiment of the control device 3 for peripheral elements 72, shown in figures 6 and 7, it comprises a plurality of buttons 301 arranged around a crown 300, either in a closed "O"-shape or an open "C"-shape, and the buttons 301 of the crown may establish a data communication link with the processing means 71. As mentioned earlier, these processing means 71 are also linked to the commutation means 73 of the peripheral elements 72 arranged in the supply tower 7.

To make it easier to handle the crown 300, it is preferably configured such that a physical link to the first end 221 of the casing 200 may be established by way of male-female coupling (see figure 6 and 7). To ensure the male-female coupling of the angulation body 20 and the crown 300, they may have any fastening means such as clips, hooks or similar.

The crown shape 300 makes the combined use of the angulation device 2 easier; the user is provide with accessible and ergonomic buttons needed to execute the orders.

Ultimately, this crown 300 must be understood as a synonym for remote control, which may be protected by a cover or bag for hygiene reasons, and may be linked to a range of endoscopes 1.

In use, the user moves the angulation portions 201, 202, 203 with respect to each other in order to insert the distal end of the endoscope 1 inside the body of the patient, following the arrows D1 and D2. With the relative movement between two of said angulation portions 201, 202, 203 the relative movement of a bending cable 204, 206 is achieved with regards to the covers 205, 207. The relative movement causes the bending portion 5 through which bending cables 204, 206 run and the covers 205, 207 to adopt the necessary angulation. One end of the bending cable 204, 206 is fixed to an angulation portion 201, 202, 203 and the other end is fixed to the distal end of the bending portion 5. The movement, for example, of the first angulation portion 201 with respect to the second portion 202 as shown in figures 2 and 3 cause the pulling or pushing of the first bending cable 204 from or towards the first covering 205 thereof, and this pulling or contraction is logically transmitted throughout the entire first bending cable 204, causing the bending portion 5 to be in an erect or curved position.

In the present invention when it has a pair of first bending cables 204, when one of them stretches, the other which is preferably situated diametrically opposed with regards to the longitudinal axis E contracts and the bending portion 5 is thus forced to adopt a certain bend.

If the user wishes to further specify a bending direction of the bending portion 5, the operation may be repeated with the relative movement between the second and third angulation portion 202, 203. All of this is carried out with one hand due to the support element 212.

Preferably but not exclusively, and the angulation body 20 seen in plan view, the arrangement of the pair of the first bending cables 204 and the pair of the second bending cables 206 represent the four cardinal points on a compass, since there is a bending cable every 90º. Similarly, it may be said that these positions enable the bending portion 5 to be bent left or right and upwards or downwards when in use.

The user may use a screen to guide themselves, such as in known endoscopes, in order to reach the objective of the endoscope and if, for example, the lens of the endoscope 1 needs to be cleaned, the user may position the fingers thereof on the predetermined marks 33, the electromyography sensors 32 read this position through the electrical impulses of the muscles, for example of the arm, and send the information to the processing means 71 which compare the reading sent by the electromyography sensors 32 with the previously stored patterns and when they detect the match between the reading and the patterns, it executes the associated order. A peristaltic pump pushes the fluid around the entire body of the endoscope 1 from the conduit 8, passing through a projection 210 and converging with the bending cables 205, 207. The fluid arrives at the distal end of the endoscope 1 through an endoscope channel (not shown) that runs through the inside of the insertion tube 4 and the bending portion 5.

In the case of using the remote control or crown 300, the user has to preferably position said crown 300 as shown in figures 6 and 7. Continuing with the angulation body 20 linked to the support element 212, the angulation body 20 is controlled with one hand and the other hand presses the buttons 301 of the crown 300 to order the activation of some of the peripheral elements 72. In this case, the processing means 71 do not need to compare the data received by a sensor with a stored pattern, given that the order from the user through the buttons leaves no room for doubt.

The details, shapes, dimensions and other accessory elements, as well as the materials used to manufacture the angulation device for an endoscope and the control device for peripheral elements of the invention may be suitably substituted for others which do not diverge from the scope defined by the claims included below.

## Claims

1. An angulation device (2) for a flexible-type endoscope (1) comprising a bending portion (5) and bending cables (204, 206) covered by covers (205, 207), **characterised by** the fact that it comprises an angulation body (20) with a long configuration and a central axis (E) arranged longitudinally along the length of said angulation body (20), wherein the angulation body (20) in turn comprises a first angulation portion (201) linked to a second angulation portion (202), said first and second angulation portion (201, 202) being articulated such that a relative back-and-forth movement therebetween is defined with regard to the central axis (E), at least one first bending cable (204) being fastened to the first angulation portion (201) and at least one first cover (205) fastened to the second angulation portion (202), such that the relative back-and-forth movement between the first angulation portion (201) and the second angulation portion (202) causes the relative movement of the first bending cable (204) with respect to the first cover (205).

2. The angulation device (2) for an endoscope (1) according to claim 1, **characterised by** the fact that the angulation body (20) further comprises a casing (200) that covers the different angulation portions (201, 202), said casing having a first end (221) by way of a handle.

3. The angulation device (2) for an endoscope (1) according to any of the preceding claims, **characterised by** the fact that it further comprises at least one third angulation portion (203) linked to the second angulation portion (202), said second and third angulation portion (202, 203) being articulated such that a relative back-and-forth movement therebetween is defined with respect to the central axis (E), at least one second bending cable (206) being fastened to the second angulation portion (202) and at least one second cover (207) fastened to the third angulation portion (203), such that the relative back-and-forth movement between the second angulation portion (202) and the third angulation portion (203) causes the relative movement of the second bending cable (206) with respect to the second cover (207).

4. The angulation device (2) for an endoscope (1) according to any of the preceding claims, **characterised by** the fact that the angulation portions (201, 202, 203) comprise hollow tubes, a pair of angulation portions being linked to each other with rods (208), the ends of which are arranged inside the angulation portions (201, 202, 203), the ends of said rods (208) being connected to the respective angulation portions (201, 202, 203) such that they rotate with respect to each other.

5. The angulation device (2) for an endoscope (1) according to claim 4, **characterised by** the fact that the hollow tubes have a square transverse cross section.

6. The angulation device (2) for an endoscope (1) according to any of claims 1-3, **characterised by** the fact that the angulation portions (201, 202, 203) are linked to each other with a spherical or ball joint (211).

7. The angulation device (2) for an endoscope (1) according to claim 6, **characterised by** the fact that the spherical or ball joint (211) is limited to one degree of freedom.

8. The angulation device (2) for an endoscope (1) according to any of the preceding claims, **characterised by** the fact that the covers (205, 207) are springs.

9. The angulation device (2) for an endoscope (1) according to any of claims 2-8, **characterised by** the fact that the casing (200) comprises a second end (233) opposite the first end, said second end (223) being provided with at least one pair of protuberances (210) that are divergent from one another, from one of which the covers (205, 207) and the bending cables (204, 206) extend.

10. The angulation device (2) for an endoscope (1) according to claim 9, **characterised by** the fact that it further comprises a support element (212) that in turn comprises at least one support surface (213) configured to coincide at least partially with the casing (200) and a projection (214) that may fit between the pair of divergent protuberances (210) when in use.

11. The angulation device (2) for an endoscope (1) according to claim 10, **characterised by** the fact that the support element (212) is linked to a stand (215).

12. A control device (3) for the peripheral elements (72) of an endoscope (1) **characterised by** the fact that it comprises a plurality of electromyography sensors (32) arranged on a band (31) made of elastic material, particularly configured as a bracelet (30) or similar, wherein the electromyography sensors (32) may establish data communication with processing means (71) and are in turn linked in data communication with at least one peripheral element (72).

13. The control device (3) for the peripheral elements (72) of an endoscope (1) according to the preceding claim, **characterised by** the fact that the band (31) is made of an elastic material.

14. The control device (3) for the peripheral elements (72) of an endoscope (1) according to any of claims 12-13 with an angulation device (2) according to any of claims 2-11, **characterised by** the fact that the casing (200) comprises predetermined marks (33) situated on the first end (221) by way of a handle.

15. The control device (3) for the peripheral elements (72) of an endoscope (1) according to the preceding claim with an angulation device (2) according to any of claims 2-11, **characterised by** the fact that the predetermined marks (33) are recesses or reliefs.

16. The control device (3) for the peripheral elements (72) of an endoscope (1) **characterised by** the fact that it comprises a plurality buttons (301) arranged around a crown (300), wherein the buttons (301) of the crown may establish data communication with processing means (71) and are in turn linked in data connection to at least one peripheral element (72).

17. The control device (3) for the peripheral elements (72) of an endoscope (1) according to the preceding claim with an angulation device (2) according to any of claims 2-11, **characterised by** the fact that the crown (300) is configured such that a link with the first end (221) of the casing (200) may be established by way of male-female coupling.

18. A supply tower (7) for an endoscope (1) **characterised by** the fact that it comprises processing means (71) and at least one peripheral element (72), wherein the peripheral element (72) in turn comprises commutation means (73) linked to the processing means (71), further comprising at least one output (74) associated with the peripheral element (72) and said output (74) being connectable to a conduit (8) of the endoscope (1) linked to an insertion tube (4); wherein the processing means (7) may be linked in data communication with a control device (3) for peripheral elements (72).

19. The supply tower (7) for an endoscope (1) according to claim 18, **characterised by** the fact that the commutation means (73) comprise a peristaltic pump, a solenoid valve or a switch.

20. The supply tower (7) for an endoscope (1) according to claim 18, **characterised by** the fact that the conduit (8) links the output (74) to the other projection (210) of the angulation device (2) for an endoscope (1) according to any of claims 9-11.
